Europäisches Patentamt

European Patent Office

Office européen des brevets

(19)

(11) Publication number: **0 184 927**
A2

# EUROPEAN PATENT APPLICATION

(12)

(21) Application number: **85308864.9**

(22) Date of filing: **05.12.85**

(51) Int. Cl.⁴: **C 01 B 33/20**, C 07 C 5/22, C 07 C 4/00, B 01 J 29/04

(30) Priority: **10.12.84 US 679775**

(43) Date of publication of application: **18.06.86**
**Bulletin 86/25**

(84) Designated Contracting States: **BE DE FR GB IT NL SE**

(71) Applicant: **MOBIL OIL CORPORATION, 150 East 42nd Street, New York New York 10017 (US)**

(72) Inventor: **Derouane, Eric Gerard, 56 Rue des Champs Verts, B-5020 Namur Champion (BE)**
Inventor: **Dessau, Ralph Moritz, 14 Celler Road, Edison, N.J. 08817 (US)**

(74) Representative: **Grundy, Derek George Ritchie et al, CARPMAELS & RANSFORD 43, Bloomsbury Square, London WC1A 2RA (GB)**

(54) **Crystalline galloborosilicates.**

(57) Galloborosilicates of zeolitic structure, Ga and B being present in the crystal lattice in defined quantity, having a low Al and alkali metal content. The materials are synthesised by vapor-phase treatment of a borosilicate zeolite with a source of gallium, and may be used as catalysts in acid-catalysed reactions such as cracking and paraffin isomerisation.

ACTORUM AG

## CRYSTALLINE GALLOBOROSILICATES

The invention relates to galloborosilicates of zeolitic structure which exhibit activity as catalysts, particularly as shape-selective cracking and aromatization catalysts, and to their preparation.

Zeolites manifest an infinitely extending three-dimensional lattice network of, conventionally, $AlO_4$ and $SiO_4$ tetrahedra linked to each other by sharing of oxygen atoms. They are conventionally represented by the empirical formula:

$$M_{2/n}O \ Al_2O_3 \ xSiO_2 \ yH_2O$$

in which x is equal to or greater than 2, and n is the valence of a cation M. The ratio of the total of silicon and aluminum atoms to oxygen atoms is 1:2. M, at least in as-synthesised or as-mined forms of the zeolite, is usually sodium, potassium, magnesium, calcium, strontium and/or barium, and establishes lattice electrical neutrality.

The prior art describes a variety of synthetic zeolites. These zeolites have come to be designated by letter or other convenient symbols, as illustrated by zeolite A (US-A-2,882,243); zeolite X (US-A-2,882,244); zeolite Y (US-A-3,130,007); zeolite ZK-5 (US-A-3,247,195); zeolite ZK-4 (US-A-3,314,752); zeolite ZSM-5 (US-A-3,709,886); zeolite ZSM-11 (US-A-3,709,979) and zeolite ZSM-23 (US-A-4,076,842), merely to name a few.

The silicon/aluminum atomic ratio of a given zeolite is often variable. For example, zeolite X can be synthesized with silicon/aluminum atomic ratios of from 1 to 1.5, zeolite Y from 1.5 to 3. In some zeolites, the upper limit of the silicon/aluminum atomic ratio is unbounded.  ZSM-5 is one such example wherein the silicon/aluminum atomic ratio is at least 2.5 and up to infinity.  US-A-3,941,871 discloses a crystalline silicate made from a reaction mixture containing no deliberately added aluminum and exhibiting the x-ray diffraction pattern characteristic of ZSM-5 zeolites.

According to the invention a crystalline galloborosilicate of zeolitic structure has a lattice comprising $SiO_4$, $GaO_4$ and $BO_4$ tetrahedra, is substantially free of alkali metal and aluminum and contains from 18 to 47% wt. silicon, no more than 46% wt. gallium and no more than 13% wt. boron.

Such a galloborosilicate can be prepared by contacting a structurally corresponding borosilicate of $SiO_2/B_2O_3$ mole ratio at least 20 with a vapour-phase source of gallium.

The invention is directed to the production of porous crystalline gallium containing borosilicates having a characteristic x-ray diffraction pattern.  Production of these gallium containing borosilicates comprises contacting a gallium compound in the vapor state with a calcined boron containing silicate reactant having a characteristic x-ray diffraction pattern, followed by treatment with an ammonium exchange solution, and subsequent calcination, to produce a porous crystalline gallium containing borosilicate in which both boron and gallium are in positions of tetrahedral substitution within the silica lattice.

The product functions as a shape selective cracking catalyst and as an aromatization catalyst.

The product contains about 18 to less than about 47 weight percent silicon; from greater than 0 to about 13 weight percent boron and from greater than 0 to about 46 weight percent gallium and is substantially free of aluminum and alkali metal; in one embodiment, the product, substantially free of aluminum and alkali metal, contains
from about 43 to less than about 47 weight percent silicon,
from greater than 0 to about 1.0 weight percent boron and
from greater than 0 to about 5.4 weight percent gallium.
Characterization of the product as substantially free of
aluminum and alkali metal means that, in quantitative
terms, the amounts of alkali metal and aluminum in the
crystalline product are desirably less than 0.1 and 0.02
weight percent, respectively. Although sources of aluminum
and alkali metal are not deliberately added as reactants in
the synthesis of the products disclosed here, their
presence, if at all, in the products is due to synthetic
techniques used to make them, as will be explained below.

The first step of the process to make these new
products involves treatment of a porous crystalline boron
containing highly siliceous material further characterized
by the fact that $^{11}B$-NMR analysis of that material
indicates the presence of tetrahedrally coordinated boron
replacing silicon in the zeolite framework. In the
examples, that material is one also having an x-ray
diffraction pattern of ZSM-5.

The preferred boron containing highly siliceous
material used as the reactant in the first step is a
calcined material crystallized from a clear liquid solution
of a source of silica, a boron oxide forming compound and
an organic compound containing an element of Group VB. No
source of aluminum or alkali metal is deliberately added to
that crystallization reaction mixture and no crystallographically significant amounts thereof appear in the
synthesized product. Aluminum and sodium are ubiquitous
and if such materials are present in minor amount in the
reactant, that presence is due to small amounts of alkali
metal and/or aluminum in the precursors of the reactant or
to impurities extracted from the reaction vessel. Thus,

F-3089                             -4-

the amounts of alkali metal and aluminum in the crystalline reactant generally can be expected to be less than 0.1 and 0.02 weight percent, respectively.

The aforementioned boron containing highly siliceous material is the crystalline, three-dimensional continuous framework silicon-containing structure which results when the oxygen atoms in the tetrahedra are shared between tetrahedral silicon or aluminum or boron atoms and which can exist with a network of essentially $SiO_2$, i.e., exclusive of intracrystalline cations. Such crystal structures comprise such materials as ZSM-5 and ZSM-12, ZSM-23, ZSM-35, ZSM-38, ZSM-48 and zeolite beta, to name but a few.

Galloborosilicates according to the invention preferably manifest a lattice $SiO_2$ to $B_2O_3$ ratio of 20 to 10,000. The organic cations, e.g., tetraalkylammonium of the freshly synthesised material, may be thermally decomposed to provide the acidic or hydrogen form of the zeolite directly without necessity for ion-exchange. The organic cations also may be replaced at least in part by other ions using conventional ion exchange techniques, although it may be necessary to calcine the as-synthesised material prior to such ion exchange. Ions introduced to replace the original organic cations may be any that are desired so long as they can pass through the channels furnished by the structure. To the extent that boron is present, it will contribute to the ion-exchange capacity of the zeolite. Desired replacing ions are metals of Groups I through VIII of the Periodic Table among which the particularly preferred are rare earth metals, manganese, zinc and those of Group VIII of the Periodic Table, e.g., platinum.

The first step in preparation of the galloborosilicate of the invention comprises preparation of a borosilicate zeolite from a reaction mixture containing a source of silica essentially free of alkali metal, an organic compound ($R_4J$) containing an element of Group V-B, preferably a quaternary ammonium compound, a boron oxide forming compound (e.g., boric acid) and water, the mixture having a composition, in terms of mole ratios of oxides, falling within the following ratios:

| REACTANTS | BROAD | PREFERRED |
|---|---|---|
| $SiO_2/B_2O_3$ | 6 to 20,000 | 10 to 10,000 |
| $M_2O/[(R_4J)_2O+M_2O]$ | 0.0 to 0.1 | 0.0 to 0.05 |
| $OH^-/SiO_2$ | 0.1 to 1.2 | 0.2 to 1.0 |
| $H_2O/[(R_4J)_2O+SiO_2]$ | 5 to 250 | 10 to 200 |
| $R_4J/SiO_2$ | 0.2 to 5 | 0.3 to 3 |

wherein $R_4J$ is as described below, M is alkali or alkaline earth metal and maintaining the mixture at crystallization temperature and at a pH between 8 and 14 until crystals of the borosilicate are formed. Since no alumina is added to the reaction mixture, the only aluminum present occurs as an impurity in some other component of the crystallization medium (with the reagents used in the examples hereinbelow, alumina impurities present resulted in less than 0.02 wt. percent aluminum in the product).

Crystallization can be carried out under either static or stirred conditions in polypropylene jars at 100°C or in stainless steel autoclaves. The useful range of temperatures is 80°C to about 180°C for about 6 hours to 150 days. Thereafter, the crystals are separated from the liquid and recovered. The composition can be prepared from materials which supply the appropriate oxide. Such compositions include oxides of silicon essentially free of alkali metal such as silicic acid, acid washed silica, tetraalkyl silicates and ammonium silicate. Boric acid, $H_3BO_3$, is the preferred source of boron although $B_2O_3$ and $H_3B_3O_6$ may also be used.

The organic compound may contain any element of Groups V-B such as nitrogen or phosphorus, preferably nitrogen. The preferred compounds are quaternary compounds wherein J is an element of Group V-B of the Periodic Table, e.g., N or P, preferably N, and each R is an alkyl or aryl group having between 1 and 7 carbon atoms, and preferably at least one R group is a methyl, ethyl, or propyl group. The oxide of the quaternary compound is generally supplied by introducing into the reaction mixture a composition such as the hydroxide, chloride or bromide of the tetraalkyl derivative of the desired V-B element, e.g., tetraethylammonium hydroxide, tetrapropylammonium hydroxide, tetrabutylphosphonium hydroxide, methyltriethylammonium chloride, and the like. Alkylammonium cation precursors generated in situ by reaction of tertiary amines with alkyl hydroxides or alkyl halides also may be used.

The quaternary compound, preferably the hydroxide, is provided in an amount sufficient to establish a pH in the crystallization reaction mixture within the range of 10 to 14, and preferably 10 to 13. If necessary, concentrated ammonium hydroxide may be added at any stage to control the pH. In a typical preparation, a silicate solution is prepared by adding a 25% tetrapropylammonium hydroxide solution in water to acid washed silica and heating to 100°C to dissolve the silica. The silicate solution is essentially free of alkali metal cations and contains less than about 60 ppm of aluminum. A boron solution prepared by mixing boric acid with 25% tetrapropylammonium hydroxide is then added to the silicate solution to provide a clear mixture which is placed in a stirred autoclave or other pressurized vessel that is maintained at a temperature of about 170°C. The mixture is then heated for 5 days to provide borosilicate crystals in which boron is tetrahedrally coordinated within the crystal lattice.
Following crystallization, the borosilicate is washed with
water and dried in a conventional manner.

Quite unexpectedly, it has been discovered that
crystallization from clear synthesis mixtures of low sodium
content, ideally none, lead to the formation of a
borosilicate having a very high degree of crystal
perfection in high yields. As shown hereinafter, in the
absence of intentionally added sodium, the crystal yield is
increased by a factor of 2. It is not fully understood why
increased yields are obtained from clear solutions of low
sodium content.

Suitable borosilicates can be prepared by the same
manipulative procedure substituting the other Group V-B
element for nitrogen. It should be realized that the oxide
can be supplied from more than one source. The reaction
mixture can be prepared either batchwise or continuously.
Crystal size and crystallization time of the new
crystalline material will vary with the nature of the
reaction mixture employed and the crystallization
conditions.

In all cases, synthesis may be facilitated by the
presence of at least 0.001 percent, preferably at least
0.10 percent and still more preferably at least 1.0
percent, seed crystals (based on total weight) of a
previously prepared crystalline product.

The borosilicate, especially in its metal, hydrogen,
ammonium alkylammonium and arylammonium forms, can be
beneficially subjected to thermal treatment. This thermal
treatment is generally performed by heating one of these
forms in an atmosphere such as air, nitrogen, hydrogen,
steam, etc., at a temperature of at least about 700°F for
at least 1 minute and generally not greater than 20 hours
to remove part or all of the water and the organic constituent. While subatmospheric pressure can be employed
for the thermal treatment, atmospheric pressure is desired
for reasons of convenience. The thermal treatment can be
performed at a temperature up to about 1700°F.

In the second step the borosilicate, preferably
substantially free of alkali metal and aluminum, is treated
with a source of gallium, under conditions to convert the
gallium source to the vapor phase. By way of example, the
gallium source may be gallium chloride. Practically, the
gallium source may be admixed with an inert gas, such as
helium or nitrogen. This step of treating the borosilicate
is usually conducted at a temperature ranging from about
100°C to about 850°C, and preferably at a temperature
ranging from about 100°C to about 500°C. The amount of
gallium source is not critical; however, the gallium source
is usually used in amounts ranging from about 0.01 to about
1 gram per gram of borosilicate. Contact times may range
from 0.05 to 12 hours. As may be expected, the amount of
gallium substituted into the boro-silicate lattice
structure can be controlled by increasing temperature
and/or increasing contact time.

Advantageously, the gallium-treated boro-silicate
material is then subjected to ammonium exchange. The
ammonium source is not critical and may be ammonium
hydroxide or an ammonium salt such as ammonium nitrate,
ammonium sulfate, or ammonium chloride. In this step, these
reagents are in aqueous solution form, such as 1N $NH_4NO_3$ or
1N $NH_4OH$. The pH of this step is of course greater than 4,
preferably about 7 to 9. Ammonium exchange may be
conducted for a period of time of from about 1 to about 20
hours at a temperature ranging from ambient to about 100°C.
Thermogravimetric analysis, following such ammonium ion
exchange, indicates the presence of two distinct ammonium
groups, one associated with framework boron and the other associated with framework gallium, both of which substitute for silicon in the crystalline silicon lattice.

The amount of ammonium associated with the galloborosilicate is about one ammonium per gallium or boron atom incorporated in the framework.

The ammonium exchanged product is calcined, typically at from about 200°C to about 600°C. Calcination may occur in an inert atmosphere of nitrogen, argon, etc. or in air at subatmospheric, atmospheric or superatmospheric pressure, from about one minute to about 48 hours.

The resulting product possesses the same crystallographic structure as that of the original borosilicate, prior to treatment with the gallium source. However, the acidic catalytic activity of the galloborosilicate is greatly increased over that of the original boro-silicate reactant.

Galloborosilicates according to the invention are characterized by catalytic activity in, inter alia, the aromatization of alkanes, e.g. n-butane and n-octane, and by an excellent selectivity for the production of para-xylenes in the aromatic fractions produced by such aromatization. Moreover, their Alpha Value is indicative of excellent catalytic cracking activity. The catalytic cracking of hydrocarbons can be undertaken under reaction conditions including a temperature of from about 300°C to about 700°C, a pressure of from about 0.1 atmosphere (BAR) to about 30 atmospheres and a weight hourly velocity of from about 0.1 to about 20. Paraffin conversion to aromatics can be effected under reaction conditions including a temperature of from about 100°C to about 700°C, a pressure of from about 0.1 atmosphere to about 60 atmospheres, a weight hourly space velocity of from about 0.5 to about 40 and a hydrogen/hydrocarbon mole ratio of from about 0 to about 20.

Catalysts comprising the galloborosilicates can be shaped in a wide variety of particle sizes. Generally speaking, the particles can be in the form of a powder, granules, or a molded product, such as an extrudate having particle size sufficient to pass through a 2 mesh (Tyler) screen and be retained on a 400 mesh (Tyler) screen. In cases where the catalyst is molded, such as by extrusion, the gallosilicate can be extruded before drying or partially dried and then extruded. It may be useful to incorporate the gallosilicate in a matrix of a material resistant to temperature and other process conditions. Such matrix material is useful as a binder and imparts greater resistance to the catalyst for the severe temperature, pressure and reactant feed stream velocity conditions encountered in many cracking processes: suitable materials are described in our EP-A-1695.

The relative proportions of the crystalline material and, for instance, inorganic oxide gel matrix, on an anhydrous basis, may vary widely with the crystalline material content ranging from between about 1 to about 99 percent by weight and more usually in the range of about 5 to about 80 percent by weight of the dry composite.

The invention is illustrated by the following nonlimiting examples. The Alpha test is described in J. Cat. 4, pp. 522-529, August 1965.

## EXAMPLES

### EXAMPLE 1

100 grams of 25% tetrapropylammonium hydroxide in water was added to 12.4 grams of acid-washed $SiO_2$ (55 ppm Al) and heated to 100°C to dissolve the silica. 2.75 grams of a solution made from 11.4 grams $H_3BO_3$ and 18 grams NaOH in 50 grams $H_2O$ was added with stirring at 80-100°C to the silicate solution, resulting in a gel, which was then heated in an autoclave for 5-1/2 days at 170°C. The yield of crystalline product was 4.9 grams.

EXAMPLE 2

A silicate solution was prepared as above. To this solution was added 2.75 grams of a solution made from 5.7 grams $H_3BO_3$ and 34 grams of tetrapropylammonium hydroxide. The resulting clear mixture was heated in an autoclave for 5 days at 170°C. The yield of crystalline product was 9.3 grams.

EXAMPLE 3

Example 2 was repeated, except that 5.5 grams of the $H_3BO_3$ solution was used. The yield of crystalline product was 10.1 grams.

EXAMPLE 4

100 grams of 20% tetraethylammonium hydroxide was added to 12.4 grams of acid-washed $SiO_2$ and heated to 100°C. To the resulting silicate solution was added 5.5 grams of a solution made from 5.7 grams $H_3BO_3$ and 34 grams of tetraethylammonium hydroxide. The clear solution was heated in an autoclave for 5 days at 170°C. The yield of crystalline product was 9.5 grams.

Examples 1 through 4 above illustrate the preparation of borosilicates having the x-ray diffraction pattern of ZSM-5. In example 1 sodium was intentionally added whereas in the synthesis mixtures of Example 2 through 4 no sodium was intentionally added. The advantage of not adding sodium is clearly seen since the yields are almost doubled and in one in one preparation, Example 3, more than doubled. Analysis by $^{11}$B-NMR indicated the presence of tetrahedrally coordinated boron replacing silicon in the
zeolite framework.  The dried product material consisted of
large (1 micron size or more) single or slightly twinned
crystals with platelet morphology.  The very high
resolution of the x-ray diffraction pattern is evidence of
very high sample crystallinity.  Scanning electron
micrograph pictures did not show contamination of the
crystalline product with gel-like impurities.  The product
analysis of Examples 1-4 are indicated in Table 1 below.

F-3089                             -13-

## TABLE 1

| SAMPLE | EX. 1 | EX. 2 | EX. 3 | EX. 4 |
|---|---|---|---|---|
| Form | As-synth. | As-synth. | As-synth. | As-synth. |
| Organic Cation | TPA[1] | TPA[1] | TPA[1] | TEA[2] |
| B (wt.%) | 0.22 | 0.21 | 0.29 | 0.48 |
| Na (wt.%) | 0.44 | 0.08 | 0.11 | 0.01 |
| Si (wt.%) | 42.06 | 30.13 | 40.38 | 46.20 |
| N (meg/g ash) | 0.68 | 0.63 | 0.62 | 0.67 |
| N (per unit cell) | 3.9 | 3.6 | 3.6 | 3.9 |
| Al (ppm) | 100 | 67 | 65 | 140 |
| $Si/B_2$ | 147 | 158 | 107 | 74 |
| $Si/Al_2$ | 8000 | 11200 | 11900 | 6300 |
| Na/B | 0.94 | 0.20 | 0.18 | 0.01 |
| Yield, grams | 4.9 | 9.3 | 10.1 | 9.5 |
| B (wt.%-via cell contraction) | 0.2-0.25 | --- | 0.3-0.35 | --- |
| N (meg/g/ash in $NH_4$-form) | 0.24 | 0.22 | 0.31 | 0.45 |
| NMR $^{11}B$ INTENSITY (relative areas):[3] | | | | |
| As-synthesized | 1.0 | 2.0 | 2.4 | 4.3 |
| $NH_4$ form | 2.0 | 2.8 | 3.8 | 7.0 |

(1)   tetrapropylammonium
(2)   tetraethylammonium
(3)   The boron-11 magic angle spinning NMR spectra were
      obtained using a JEOL FX-200 Fourier Transform
      Spectrometer equipped with a Chemagnetics solid state NMR
      variable temperature broadband magic angle spinning
      probe.  Spectra were obtained using 8-64 90° (4.5  s)
      pulses at 3  s intervals.  Spinning rates ranged from
      3.8-4.2 KHz.  Spectra were digitized with 8K data points
      (no zero filling over a 32 KHz (500 ppm) spectral width.
      Gated high power proton decoupling was used on the spectra.

## EXAMPLE 5

A 2.31g sample of the calcined product of Example 4 was treated with about 1.3g of gallium chloride vapors in a 2-zone treatment, lasting about 3 hours; specifically, the calcined borosilicate of Example 4 was maintained at 300°C, while the gallium chloride was vaporized at 150°C. The temperature was then increased to 500°C. The product was subjected to ammonium ion exchange at a pH of 9 using a solution of 1M $NH_4Cl/NH_4OH$ at room temperature overnight. Thermogravimetric analysis following ammonium ion exchange, indicated the presence of two distinct ammonium groups, one associated with framework boron and the other associated with framework gallium. The TGA was conducted by heating the sample 20°C/minute up to a temperature of about 700°C. About 0.25 meq of $NH_3$ desorbed at temperatures below 300°C; and about 0.15 meq of $NH_3$ evolved at a temperature above 300°C. All ammonium ion exchanged at sites provided by boron contained in the framework of a silicate zeolite will evolve, i.e., desorb, at temperatures below 300°C. The total ammonium content was 0.40 meq/g. The ammonium ion exchanged product was subjected to calcination at a temperature of about 500°C.

Elemental analysis of the calcined product indicated that the product contained 1.06 weight percent Ga (0.15 meq/g) and 0.37% B (0.34 meq/g). Together with the foregoing ammonium ion exchange data, the results of the elemental analysis indicates predominant incorporation and substitution of gallium and boron for silicon in the silica lattice of the crystallographic structure corresponding to ZSM-5.

## EXAMPLE 6

The hydrogen form of the product of Example 5, produced by calcination, is an effective catalyst for
hexane cracking as indicated by its initial alpha values:
$alpha_{5\ min}$ = 90; $alpha_{45\ min.}$ = 50 and $alpha_{120\ min.}$ = 36.
By comparison, initial alpha value for the borosilicate
starting material of Example 5, prior to gallium
incorporation, was less than 1.

### EXAMPLE 7

Butane dehydrogenation and aromatization was conducted
employing 958 mg of the product of Example 5 at an n-butane
flow rate of about 60cc/min at a temperature of about
1000°F (538°C). After 75 minutes, the butene selectivity
of the catalyst was determined to be 19%; and its BTX
selectivity, 31%. Hydrogen production was measured at
greater than one mole per mole of butane conversion. This
high hydrogen production indicates the dehydrogenation and
high aromatization activity of such Ga-containing
catalysts. Gas chromatography results indicated that 80%
of the xylenes produced in this example were p-xylenes.

### EXAMPLE 8

In this experiment, 0.884g of the galloborosilicate of
Example 5 was tested for its capacity to catalyze
aromatization of n-octane. The result of feeding n-octane
at 1 ml/hr, nitrogen at 22 cc/min over the catalyst at a
temperature of about 1000°F 538°C) was a yield of 40-50%
BTX at 95-99% octane conversion. Furthermore, the
selectivity to para-xylene in the xylene isomers was 75%,
indicative of shape selectivity.

F-3089                                    -16-

# CLAIMS

1.  A crystalline galloborosilicate of zeolitic structure having a lattice comprising $SiO_4$, $GaO_4$ and $BO_4$ tetrahedra, said galloborosilicate being substantially free of alkali metal and aluminum and comprising from 18 to 47% wt. silicon, no more than 46% wt. gallium and no more than 13% wt. boron.

2.  A galloborosilicate according to claim 1 which comprises at least 43% wt. silicon, no more than 5.4% wt. gallium and no more than 1.0% wt. boron.

3.  A galloborosilicate according to claim 1 or claim 2 which contains no more than 0.1% wt. alkali metal.

4.  A galloborosilicate according to any preceding claim which contains no more than 0.02% wt. aluminum.

5.  A galloborosilicate according to any preceding claim which manifests the x-ray diffraction data of zeolite ZSM-5, -12, -23, -35, -38, -48 or beta.

6.  A galloborosilicate according to any preceding claim which is in the hydrogen form.

7.  A method of preparing a galloborosilicate as claimed in claim 1 which comprises contacting a borosilicate zeolite of $SiO_2/B_2O_3$ mole ratio of at least 20 with a vapor-phase source of gallium until the zeolite contains no more than 46% wt. gallium.

8. A method according to claim 7 wherein the product of the contacting is ammonium exchanged and then calcined to produce the hydrogen form of the galloborosilicate.

9. A method according to claim 7 or claim 8 wherein said borosilicate is substantially free of alkali metal and aluminum.

10. A method according to any of claims 7 to 9 wherein the contacting is performed at a temperature of 100 to 850°C.

11. A method according to any of claims 7 to 9 wherein the contacting is performed at a temperature of 100 to 500°C.

12. A method according to any of claims 7 to 11 wherein the source of gallium is gallium chloride.

13. A method according to any of claims 7 to 12 wherein the weight ratio of gallium source to borosilicate during the contacting is 0.01 to 1.

14. A method according to any of claims 7 to 13 wherein the contact time between gallium source and borosilicate is 0.05 to 12 hours.

15. A method according to any of claims 7 to 14 wherein during the contacting the source of gallium is in admixture with an inert gas.

16. A method according to any of claims 7 to 15, wherein the borosilicate is formed by crystallization from a reaction mixture containing a source of silica essentially free of alkali metal, a compound $R_4J$ in which R
is an alkyl of 1 to 7 carbon atoms or aryl and J is N or P,
a boron oxide forming compound and water, said reaction
mixture having the composition in terms of mole ratios of
oxides:

| | |
|---|---|
| $SiO_2/B_2O_3$ | 6 to 20,000 |
| $M_2O/[(R_4J)_2O+M_2O]$ | 0.0 to 0.1 |
| $OH^-/SiO_2$ | 0.1 to 1.2 |
| $H_2O/[(R_4J)_2O+SiO_2]$ | 5 to 250 |
| $R_4J/SiO_2$ | 0.2 to 5 |

17.  A method according to claim 16 wherein the
respective mole ratios are:

| | |
|---|---|
| $SiO_2/B_2O_3$ | 10 to 10,000 |
| $M_2O/[(R_4J)_2O+M_2O]$ | 0.0 to 0.05 |
| $OH^-/SiO_2$ | 0.2 to 1.0 |
| $H_2O/[(R_4J)_2O+SiO_2]$ | 10 to 200 |
| $R_4J/SiO_2$ | 0.3 to 3 |

18.  A method according to claim 16 or claim 17
wherein $R_4J$ is tetraethylammonium or tetrapropylammonium.

19.  A method according to any of claims 16 to 18
wherein the reaction mixture is a clear liquid solution.

20.  A method according to claim 19 wherein the
reaction mixture is assembled by mixing a solution of the
source of silica in aqueous $R_4J$ with a solution of the
boron oxide forming compound in aqueous $R_4J$.

21. Use of galloborosilicates as claimed in any of claims 1 to 6 as catalyst or catalyst components in acid-catalysed hydrocarbon conversion processes.

22. Use according to claim 21 wherein the conversion process is aromatisation of paraffins, or cracking.